# EUROPÄISCHE PATENTSCHRIFT

(11) **EP 2 801 344 B1**
(45) Veröffentlichungstag und Bekanntmachung des Hinweises auf die Patenterteilung: **10.01.2018**
(21) Anmeldenummer: 14001620.5
(22) Anmeldetag: 08.05.2014
(51) Int. Cl.: A61F 13/49, A61F 13/496

(54) **Saugfähiger Einweg-Artikel**
Disposable absorbent article
Article à usage unique absorbant

(30) Priorität: 10.05.2013 PL 40384413
(43) Veröffentlichungstag der Anmeldung: 12.11.2014
(73) Patentinhaber: TZMO S.A., 87 - 100 Torun (PL)
(72) Erfinder: Przybylski, Tomasz, 87-300 Brodnica (PL); Cwiertnia, Magdalena, 87-100 Torun (PL); Rychlik, Joanna, 87-300 Brodnica (PL)
(74) Vertreter: Bischof, Oliver

(56) Entgegenhaltungen:
- EP-A1- 2 241 296
- EP-B1- 0 970 677
- WO-A1-2007/037391
- JP-A- 2011 234 847
- US-A- 5 074 854

## Beschreibung

Die Erfindung betrifft einen saugfähigen Einweg-Artikel in Höschenform, bestehend aus einem vorderen Teil, einem hinteren Teil und einem zwischen diesen Teilen angeordneten Schrittteil, wobei das vordere Teil und das hintere Teil mit elastischen Elementen versehen sind, die während des Gebrauchs des Artikels die Taille und die Hüften des Trägers umgeben und miteinander mit der Hilfe von seitlichen Schweißnähten verbunden sind und Öffnungen für die Taille und für die Beine des Benutzers bilden, wobei das vordere Teil und das hintere Teil des Artikels zusätzlich zur Fixierung der elastischen Taillenelemente und elastischen Hüftelemente dienende Befestigungsschweißnähte aufweisen.

Ein saugfähiger Einweg-Artikel der im Oberbegriff angegebenen Art lässt sich der JP 2011 234 847 A entnehmen. Bei dem bekannten Einweg-Artikel sind zusätzliche Befestigungsschweißnähte vorgesehen, welche als unterschiedliche Zugfestigkeit aufweisende Abreißelemente konzipiert sind, mit denen sich die Windel von dem Körper des Benutzers einfacher entfernen lässt. Die Abreißelemente verlaufen parallel zu den seitlichen Schweißnähten. Nachteilig ist, dass die zusätzlichen Befestigungsschweißnähte alle Materialschichten durchdringen und reichen bis zu einer Windelinnenfläche, so dass Hautreizungen am Körper des Benutzers auftreten können.

Saugfähige Einweg-Artikel in Höschenform sind bekannt und weit verbreitet. Solche Artikel weisen zahlreiche Elastifizierungsmittel auf, gewöhnlich in Form von elastischen Gummifäden, die im vorgespannten Zustand mit den Stoff-Materialien des Einweg-Artikels verbunden werden, vorzugsweise durch Verkleben. Bei bekannten Artikeln umgibt im vorderen Teil und hinteren Teil ein elastifiziertes Band die Taille und die Hüften des Benutzers. Bei derartigen Artikeln kommen elastische Elemente auch in seitlichen Sperrelementen vor, die an einer Absorptionseinlage und um die jeweiligen Beinöffnungen herum angeordnet sind.

Die elastischen Elemente im Bereich der Taille bzw. Hüfte haben meistens die Form von mehreren Fäden oder Gummibändern, die durchgehend das Taillen- und Hüftteil des Artikels umfassen und deren Funktion vor allem darin besteht, den Artikel am Körper seines Benutzers zu fixieren. Das Verhindern des Herunterrutschens des saugfähigen Artikels vom Körper des Benutzers während des Gebrauchs hängt vor allem von einer entsprechenden Konstruktion des Taillen- bzw. Hüft-Bandes und von einer richtigen Anordnung von elastischen Elementen in den dafür vorgesehenen Bereichen ab. Damit die elastischen Elemente ihre Andrück-Funktion erfüllen können, werden sie mit Hilfe von Klebstoff im vorgespannten Zustand befestigt. Darauf folgt ihre Entspannung, was dazu führt, dass das Stoffmaterial im fertigen Artikel sich wellt und zusammenläuft, wodurch Verformungen, Falten, Wellen und lokale Verdickungen entstehen.

Die elastischen Elemente des Taillen-Hüftbereiches können, auch wenn sie an die Stoffschichten angeklebt sind, ihre Lage ändern; es ist das so genannte Phänomen des "Kriechens von Gummibändern". In Folge dessen können die elastischen Elemente ihre Andrück-Funktionen beim Gebrauch des Artikels nicht mehr erfüllen, wodurch der Artikel am Körper des Trägers abrutscht und herunterfällt.

Um elastische Elemente besser zu fixieren, ist es bekannt, Taillen-Gummibänder und Hüft-Gummibänder in einer seitlichen Schweißnaht anzuordnen, die das vordere Teil des Artikels mit seinem hinteren Teil verbindet. Zusätzlich sind elastische Elemente in diesem Bereich der seitlichen Schweißnaht angeklebt, damit sie nicht verrutschen. Die Anwendung einer solchen Lösung führt dazu, dass die Taillen- und Hüft-Gummibänder aus dem vorderen Teil und hinteren Teil sich in der seitlichen Schweißnaht überlappen, wodurch es schwierig wird, diesen Bereich zu verschweißen. Die Folge ist, dass die elastischen Elemente aus den Schweißnähten austreten, denn das Verschweißen einer zu dicken Schicht von Stoffen in einer geeigneten Art und Weise ist unmöglich. Das Resultat ist, dass das elastische Band seine Andrück-Funktion nicht erfüllt und der Artikel während des Tragens von Körper des Trägers herunterrutscht. Die zusätzliche Verankerung von Taillen- und Hüft-Gummibändern in den seitlichen Schweißnähten mit Zusatz von Klebstoff bewirkt, dass die Schweißnähte steif werden und sich unangenehm anfühlen. Dies kann beim Benutzer zu Hautreizungen und Schürfungen während des Gebrauchs des Artikels führen. Eine andere ungünstige Folge des Vorhandenseins von Klebstoff in der seitlichen Schweißnaht ist das Abschwächen ihrer Kraft, was die Funktionalität des Artikels wesentlich beeinflusst. Kraftwerte unter 5 N pro Band verursachen spontanes Reißen der Schweißnaht bereits während des Anziehens (Anlegens) des Artikels durch seinen Benutzer.

Um das Problem des Austretens von Gummibändern aus den seitlichen Schweißnähten zu vermeiden, wird in der Patentschrift EP-B1-0 787 474 vorgeschlagen, die elastischen Elemente des Taillen-Hüftbereiches im vorderen und hinteren Teil so anzuordnen, damit sie sich an der Verschweißungsstelle nicht überlappen, sie also abwechselnd anzuordnen (sog. "interposed"). Eine solche Anordnung von Gummibändern erlaubt zwar, das vordere Teil und das hintere Teil ziemlich genau miteinander zu verschweißen, jedoch kann der Effekt des sog. "Kriechens von Gummibändern" immer noch auftreten. Die seitliche Schweißnaht bleibt weiterhin steif und fühlt sich unangenehm an, was zu Hautirritationen und Abschürfungen beim Tragen des Artikels führt.

Die Patentschrift EP-B1-0 970 677 offenbart einen saugfähigen Artikel, der nicht nur seitliche Schweißnähte, sondern auch zusätzliche Schweißnähte aufweist, die alle Schichten des Taillen-Hüftbereiches in dem vorderen und hinteren Teil miteinander verbinden. Die Aufgabe dieser Erfindung sei es, dem Auseinandergehen dieser Schichten während des Aufreißens der seitlichen Schweißnähte entgegenzuwirken, damit der Inhalt des Artikels nicht nach außen gelangt. Das vorgeschlagene Verschweißen sämtlicher Schichten des Taillen-Hüftbereiches an vier zusätzlichen Stellen hat aber zur Folge, dass der Benutzer mit Hautreizungen und Schürfungen rechnen muss, was die Qualität des Artikels mindert. Ein saugfähiger Artikel gemäß WO 2007/037391 A weist Perforationslinien auf, welche sich am Vorderteil des Einweg-Artikels befinden und parallel zu seitlichen Schweißnähten verlaufen. Die Perforationslinien ermöglichen - nach Bedarf - das Abreißen des Vorderteils und Wechseln des Höschens. Bei einem Inkontinenzartikel gemäß EP 2 241 296 A1 sind relativ breite Fugenbereiche zu sehen, welche im Überlappungsbereich des Schrittabschnitts mit dem Bauch- bzw. Rückenabschnitt angeordnet sind. Die Aufgabe der Fugenbereiche ist, eine reißfeste Verbindung des Schrittabschnitts mit dem Bauch- bzw. Rückenabschnitt zu bilden. Ferner zeigt US 5 074 854 A ein wegwerfbares Höschen mit seitlichen Abreißfeldern, welche jeweils durch zwei voneinander beabstandete Nähte gebildet sind, wobei das Abreißfeld eine geringere Reißfestigkeit als die der Nähte aufweist, so dass ein schnelles Wechseln des Höschens durch den Benutzer, wie bei vorgenannter Schrift WO 2007/037391 A möglich ist.

Die Aufgabe der vorliegenden Erfindung besteht darin, die Unannehmlichkeiten, insbesondere die Möglichkeit des Auftretens von Hautreizungen oder Schürfungen zu eliminieren, den Tragekomfort des Benutzers des saugfähigen Artikels zu verbessern, sowie dem sog. Effekt des "Kriechens von Gummibändern" entgegenzuwirken, was zur besseren Anpassung des Artikels an den Körper des Benutzers führt und bewirkt, dass der Artikel nicht herunterrutscht.

Zur Lösung der genannten Aufgabe wird ein saugfähiger Einweg-Artikel vorgeschlagen, bei dem das vordere Teil und das hintere Teil des Artikels zusätzlich zur Fixierung der elastischen Taillenelemente und elastischen Hüftelemente dienende Befestigungsschweißnähte aufweisen, wobei die Befestigungsschweißnähte eine äußere Vliesstoffschicht eines Taillen-Hüftbereiches sowie wenigstens teilweise die Taillenelemente und die Hüftelemente durchdringen und damit die äußere Vliesstoffschicht und die elastischen Elemente des vorderen Teils und des hinteren Teils miteinander verbinden, so dass keinen Kontakt mit der Schicht haben, welche - im benutzten Zustand - direkt an der Haut des Benutzers anliegt.

Die zusätzlichen Befestigungsschweißnähte dienen zum Fixieren von elastischen Taillen- und Hüftelementen. Die Befestigungsschweißnähte verlaufen durch eine äußere Vliesstoffschicht und durch elastische Elemente des vorderen Teils und des hinteren Teils. Auf diese Weise wird das Verlagern dieser elastischen Elemente während des Gebrauchs des Artikels verhindert. Die übrigen Vliesstoffschichten des Taillen-Hüftbereiches befinden sich außerhalb des Bereichs dieser zusätzlichen Befestigungsschweißnähte. Die Befestigungsschweißnähte reichen nicht bis zur Schicht, die einen direkten Kontakt mit der Haut des Benutzers hat. Die Aufgabe von Befestigungsschweißnähten besteht nur darin, die Taillen- und Hüft-Gummibänder festzuhalten und somit dem sog. Effekt des "Kriechens von Gummibändern" entgegenzuwirken.

Die vorliegende Erfindung stellt einen voll funktionsfähigen Hygiene-Artikel zur einmaligen Verwendung in der Form eines saugfähigen Höschens dar. Dank der Verwendung von zusätzlichen Schweißnähten im Bereich des Taillen-Hüftbereiches ist es möglich, die elastischen Elemente aus den seitlichen Schweißnähten zurückzuziehen, wodurch die seitlichen Schweißnähte weniger steif werden. Eine solche Konstruktionsweise wirkt Hautirritationen und Schürfungen entgegen, was die Funktionstüchtigkeit des Artikels wesentlich erhöht.

Der hauptsächliche Vorteil jedoch, den die Anwendung solcher Befestigungsschweißnähte mit sich bringt, besteht in dem Entgegenwirken der Verlagerung von Taillen- und Hüft-Gummibändern. Dies hat einen wesentlichen Einfluss auf die richtige Anpassung des Artikels an den Körper des Trägers und wirkt dem Herunterrutschen des Artikels entgegen. Die Verwendung von zusätzlichen Befestigungsschweißnähten mindert den Tragekomfort des Artikels nicht. Die Befestigungsschweißnähte verursachen auch keine Hautreizungen, denn sie verlaufen durch eine äußere Schicht und haben keinen Kontakt mit der Schicht, welche direkt an der Haut des Benutzers anliegt.

Der Gegenstand der Erfindung ist in einem Ausführungsbeispiel anhand der Zeichnung veranschaulicht, deren Figuren zeigen:
- Fig. 1: einen Einweg-Hygiene-Artikel in der Form eines saugfähigen Höschens mit zusätzlichen Befestigungsschweißnähten, in zusammengefügtem Zustand, d.h. mit seitlichen Schweißnähten;
- Fig. 1a: ein vergößertes Detail der Fig. 1, mit besonderer Berücksichtigung der Befestigungsschweißnähte und einer seitlichen Schweißnaht,
- Fig. 2: einen Zuschnitt für einen Hygiene-Artikel gem. Fig. 1 mit zusätzlichen Befestigungsschweißnähten, in der Sicht auf die Außenseite, d.h. die Seite, die keinen Kontakt mit der Haut des Trägers hat.

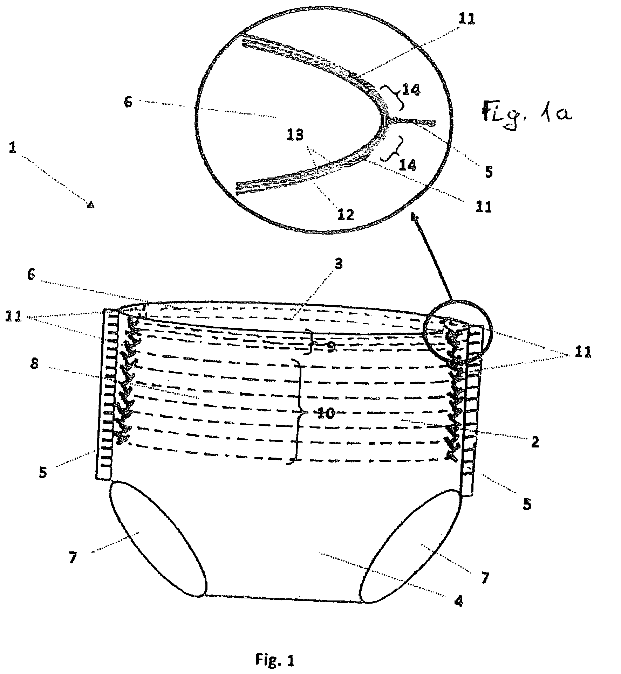

Fig. 1 zeigt ein Ausführungsbeispiel eines Einweg-Artikel 1 in Form eines saugfähigen Höschens, bestehend aus einem vorderen Teil 2, einem hinteren Teil 3 und einem Schrittteil 4. Das vordere Teil 2 und das hintere Teil 3 sind mit der Hilfe von seitlichen Schweißnähten 5 miteinander verbunden. Die seitlichen Schweißnähte 5 sind als Ultraschallschweißnähte ausgeführt und bilden eine Taillenöffnung 6 und Beinöffnungen 7.

Ein Taillen-Hüftbereich 8,der über die Vorder- und Rückseite des Einweg-Artikels 1 verläuft, weist elastische Taillenelemente 9 in seinem oberen Bereich und elastische Hüftelemente 10 in seinem unteren Bereich auf, die parallel zur Öffnungskante 18 der Taillenöffnung verlaufen. Die elastischen Taillenelemente 9 und Hüftelemente 10 bestehen aus elastischem Garn. Die elastischen Taillenelemente 9 und Hüftelemente 10 sind nicht im Bereich der seitlichen Schweißnaht 5 angeordnet.

Zur Fixierung der elastischen Taillenelemente 9 und Hüftelemente 10 dienen Befestigungsschweißnähte 11, die im vorderen Teil 2 und im hinteren Teil 3 verlaufen. Wie Fig. 1a zeigt, sind sie um den Abstand 14 von den seitlichen Schweißnähten 5 beabstandet. Die zur Fixierung der elastischen Taillenelemente 9 und Hüftelemente 10 dienenden Befestigungsschweißnähte 11 durchdringen eine äußere Vliesstoffschicht 12 des Taillen-Hüftbereiches 8, sowie weiggstens teilweise die Taillenelemente 9 und die Hüftelemente 10. Durch die übrigen Vliesstoffschichten 13 des Taillen-Hüftbereiches 8 verlaufen die Befestigungsschweißnähte 11 nicht.

Die Befestigungsschweißnaht 11 ist eine Ultraschallschweißnaht. Die Befestigungsschweißnähte 11 befinden sich in einem gewissen Abstand 14 von den seitlichen Schweißnähten 5. Zwischen den seitlichen Schweißnähten 5 und den Befestigungsschweißnähten 11 können sich Enden der elastischen Taillenelemente 9 und Hüftelemente 10 befinden.

Die Befestigungsschweißnähte 11 verlaufen durch den Endbereich der Taillenelemente 9 und Hüftelemente 10. Es gibt je zwei Befestigungsschweißnähte 11 am vorderen Teil 2 und hinteren Teil 3 in der Nähe der Schweißnähte 5. Die Zahl der Befestigungsschweißnähte 11 am vorderen Teil 2 und hinteren Teil 3 kann größer als 2 sein, d.h. es können weitere Befestigungsschweißnähte 11 im Bereich des Taillen-Hüftbereiches 8 parallel zueinander angeordnet sein. Zur Vereinfachung sind in Fig. 1 nur ein vorderes und ein hinteres Paar gezeigt.

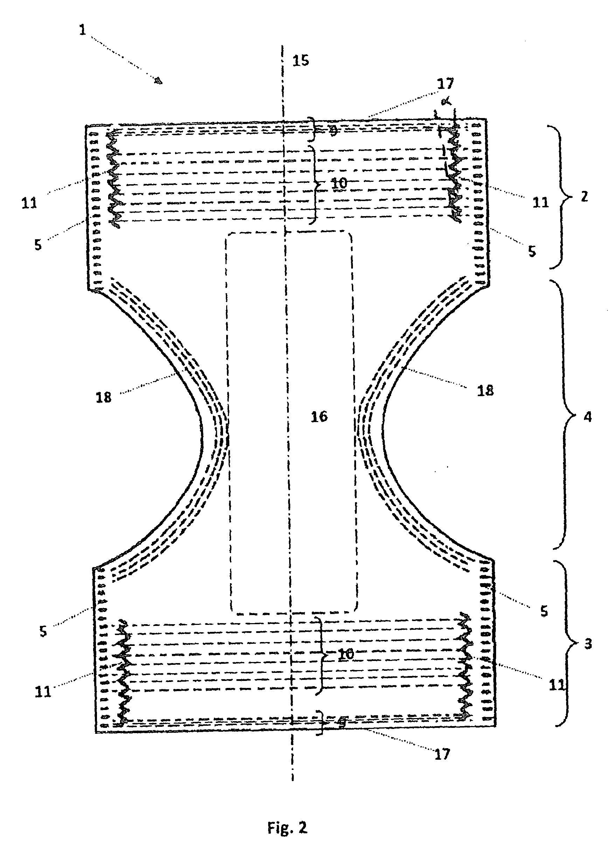

Fig. 2 zeigt einen Zuschnitt des Einweg-Artikels 1 in aufgeklapptem Zustand. Der Zuschnitt ist in der Sicht auf die Außenseite dargestellt, d.h. auf die Seite, die keinen Kontakt mit der Haut des Trägers hat. Andeutungsweise ist die auf der Innenseite liegende Absorptionseinlage dargestellt.

Die Befestigungsschweißnähte 11 verlaufen im dargestellten Ausführungsbeispiel parallel zur Längsachse 15 des Artikels. Alternativ können die Befestigungsschweißnähte 11, unter einem gewissen Winkel α, beispielsweise von ca. 5°, zur Längsachse 15 des Artikels verlaufend angeordnet sein.

Aus der Fig. 2 geht weiterhin hervor, dass die Befestigungsschweißnähte 11 durch einen Bereich verlaufen, in dem sich keine Absorptionseinlage 16 befindet. Auch die elastischen Taillenelementen 9 und Hüftelementen 10 verlaufen nicht durch die Absorptionseinlage. Es kann jedoch bei einer anderen Ausführungsform die Absorptionseinlage 16 von den elastischen Taillenelementen 9 und Hüftelementen 10 überlappt sein. In einem solchen Fall können die Befestigungsschweißnähte 11 teilweise durch den Bereich verlaufen, in dem die Absorptionseinlage 16 angeordnet ist.

Die Befestigungsschweißnähte 11 verlaufen von der Öffnungskante 17 der Taillenöffnung 6 beginnend bis zum Ende des Taillen-Hüftbereichs 8, in dem elastische Taillenelemente 9 und Hüftelemente 10 angeordnet sind. Alternativ können die zur Fixierung von den elastischen Taillenelementen 9 und Hüftelementen 10 dienenden Befestigungsschweißnähte 11 von der Kante 17 der Taillenöffnung 6 bis zur Beinöffnung 7 verlaufen. In diesem Falle werden auch die elastischen Elemente 18 der Beinöffnung 7 mit verschweißt.

### Bezugszeichenliste:

- 1: Einweg-Artikel
- 2: vorderes Teil
- 3: hinteres Teil
- 4: Schrittteil
- 5: seitliche Schweißnaht
- 6: Taillenöffnung
- 7: Beinöffnung
- 8: Taillen-Hüftbereich
- 9: elastische Taillenelemente
- 10: elastische Hüftelemente
- 11: Befestigungsschweißnaht
- 12: äußere Vliesstoffschicht
- 13: (weitere) Vliesstoffschichten
- 14: Abstand
- 15: Längsachse
- 16: Absorptionseinlage
- 17: Öffnungskante
- 18: elastische Elemente
- α: Winkel

## Patentansprüche

1. Saugfähiger Einweg-Artikel in Höschenform, bestehend aus einem vorderen Teil, einem hinteren Teil und einem zwischen diesen Teilen angeordneten Schrittteil, wobei das vordere Teil und das hintere Teil mit elastischen Elementen versehen sind, die während des Gebrauchs des Artikels die Taille und die Hüften des Trägers umgeben und die miteinander mit der Hilfe von seitlichen Schweißnähten verbunden sind und Öffnungen für die Taille und für die Beine des Benutzers bilden, wobei das vordere Teil (2) und das hintere Teil (3) des Artikels (1) zusätzlich zur Fixierung der elastischen Taillenelemente (9) und elastischen Hüftelemente (10) dienende Befestigungsschweißnähte (11) aufweisen,
**dadurch gekennzeichnet, dass**
die Befestigungsschweißnähte (11) eine äußere Vliesstoffschicht (12) eines Taillen-Hüftbereiches (8) sowie wenigstens teilweise die Taillenelemente (9) und die Hüftelemente (10) durchdringen und damit die äußere Vliesstoffschicht (12) und die elastischen Elemente (9, 10) des vorderen Teils (2) und des hinteren Teils (3) miteinander verbinden, so dass keinen Kontakt mit der Schicht haben, welche - im benutzten Zustand - direkt an der Haut des Benutzers anliegt.

2. Saugfähiger Einweg-Artikel nach Anspruch 1, **dadurch gekennzeichnet, dass** die zur Fixierung von den elastischen Taillenelementen (9) und Hüftelementen (10) dienenden Befestigungsschweißnähte (11) unter einem Winkel (α) zur Längsachse (15) des Artikels (1) verlaufen.

3. Saugfähiger Einweg-Artikel nach Anspruch 1, **dadurch gekennzeichnet, dass** die zur Fixierung von den elastischen Taillenelementen (9) und Hüftelementen (10) dienenden Befestigungsschweißnähte (11) parallel zu einer Längsachse (15) des Artikels (1) verlaufen.

4. Saugfähiger Einweg-Artikel nach Anspruch 1,**dadurch gekennzeichnet, dass** im vorderen Teil (2) und hinteren Teil (3) des Artikels (1) sich je zwei Befestigungsschweißnähte (11) befinden, die von den seitlichen Schweißnähten (5) beabstandet sind, wobei der Abstand von den seitlichen Schweißnähten (5) vorzugsweise nicht weniger als 1 mm beträgt.

## Claims

1. Disposable absorbent article in the form of pants, consisting of a front part, a rear part and a crotch part arranged between these parts, wherein the front part and the rear part are provided with elastic elements which, during use of the article, go around the waist and the hips of the wearer, are connected to one another with the aid of lateral weld seams and form openings for the waist and for the legs of the user, wherein the front part (2) and the rear part (3) of the article (1) additionally have fastening weld seams (11) serving to fix the elastic waist elements (9) and elastic hip elements (10),
**characterised in that**
the fastening weld seams (11) penetrate an outer non-woven material layer (12) of a waist-hip region (8) and penetrate at least partially the waist elements (9) and the hip elements (10) and thus connect the outer non-woven material layer (12) and the elastic elements (9, 10) of the front part (2) and of the rear part (3) to one another so that they do not have any contact with the layer which - in the used state - lies directly against the skin of the user.

2. Disposable absorbent article as claimed in claim 1, **characterised in that** the fastening weld seams (1) serving to fix the elastic waist elements (9) and hip elements (10) extend at an angle (α) to the longitudinal axis (15) of the article (1).

3. Disposable absorbent article as claimed in claim 1, **characterised in that** the fastening weld seams (11) serving to fix the elastic waist elements (9) and hip elements (10) extend in parallel with a longitudinal axis (15) of the article (1).

4. Disposable absorbent article as claimed in claim 1, **characterised in that** two fastening weld seams (11) which are spaced apart from the lateral weld seams (5) are each located in the front part (2) and rear part (3) of the article (1), wherein the spacing from the lateral weld seams (5) is preferably not less than 1 mm.

## Revendications

1. Article absorbant à usage unique présentant une forme de culotte, constitué d'une partie avant, d'une partie arrière et d'une partie d'entrejambe disposée entre lesdites parties, dans lequel la partie avant et la partie arrière sont pourvues d'éléments élastiques, qui entourent, lors de l'utilisation de l'article, la taille et les hanches du porteur et qui sont reliés les uns aux autres à l'aide de cordons de soudure latéraux et qui forment des ouvertures pour la taille et pour les jambes de l'utilisateur, dans lequel la partie avant (2) et la partie arrière (3) de l'article (1) présentent en supplément des cordons de soudure de fixation (11) servant à la fixation des éléments de taille (9) élastiques et des éléments de hanche (10) élastiques,
**caractérisé en ce que**
les cordons de soudure de fixation (11) traversent une couche en non-tissé (12) extérieure d'une zone de taille et de hanche (8) ainsi qu'au moins en partie les éléments de taille (9) et les éléments de hanche (10) et relient ainsi la couche en non-tissé (12) extérieure et les éléments (9, 10) élastiques de la partie avant (2) et de la partie arrière (3) les uns aux autres de sorte qu'ils n'ont aucun contact avec la couche qui repose - dans l'état utilisé - directement contre la peau de l'utilisateur.

2. Article absorbant à usage unique selon la revendication 1, **caractérisé en ce que** les cordons de soudure de fixation (11) servant à la fixation des éléments de taille (9) et des éléments de hanche (10) élastiques s'étendent selon un angle (α) par rapport à l'axe longitudinal (15) de l'article (1).

3. Article absorbant à usage unique selon la revendication 1, **caractérisé en ce que** les cordons de soudure de fixation (11) servant à la fixation des éléments de taille (9) et des éléments de hanche élastiques s'étendent de manière parallèle par rapport à un axe longitudinal (15) de l'article (1).

4. Article absorbant à usage unique selon la revendication 1, **caractérisé en ce que** se trouvent dans la partie avant (2) et dans la partie arrière (3) de l'article (1) respectivement deux cordons de soudure de fixation (11), qui sont espacés des cordons de soudure (5) latéraux, dans lequel l'espacement par rapport aux cordons de soudure (5) latéraux n'est de préférence pas inférieur à 1 mm.
